# EUROPEAN PATENT APPLICATION

(11) **EP 0 924 033 A2**
(43) Date of publication of application: **23.06.1999**
(21) Application number: 98310231.0
(22) Date of filing: 14.12.1998
(51) Int. Cl.: B25J 9/10

(54) **Artificial muscles**

(30) Priority: 15.12.1997 JP 34481797; 15.12.1997 JP 34481897
(71) Applicant: Kaneto, Keiichi, Fukuoka-shi, Fukuoka 810-0062 (JP); Kaneka Medix Corporation, Osaka-shi, Osaka 530-8288 (JP)
(72) Inventor: Kaneto, Keiichi, Fukuoka-shi, Fukuoka 810-0062 (JP); Sewa, Shingo, Ashigarakami-gun, Kanagawa 259-0151 (JP); Takashima, Wataru, Kitakyushu-shi, Fukuoka 807-1131 (JP)
(74) Representative: Skailes, Humphrey John

(57) **Abstract**

The present invention provides an artificial muscle comprising a solid electrolyte molded product (2) having a shape of a slender rectangular flat plate and a pair of polyaniline membranes (3a,3b) which are formed on both surfaces of the solid electrolyte molded product (2) and are insulated from each other, said artificial muscle being designed so that it can be freely deformed in the given direction by applying a voltage to the polyaniline membranes (3a,3b) to deform the polyaniline membranes. The present invention further provides an artificial muscle comprising a solid electrolyte molded product (2) having a shape of a slender rectangular flat plate, a pair of polyaniline membranes (3a,3b) which are formed on both surfaces of the solid electrolyte molded product (2) and are insulated from each other, and metallic electrodes (4a,4b) provided on the polyaniline membranes, said artificial muscle being designed so that it can be freely deformed in the given direction by applying a voltage between the metallic electrodes (4a,4b) to deform the polyaniline membranes. The artificial muscles are flexible and lightweight, have quick response and are capable of undergoing large deformation.

## Description

### FIELD OF THE INVENTION

The present invention relates to artificial muscles, and more particularly to artificial muscles comprising polyaniline membranes, which are flexible, are capable of being driven at a low voltage, have great shrinkage force and are capable of retaining positions.

### BACKGROUND OF THE INVENTION

Artificial muscles hitherto known include an electrostatic attraction actuator, a piezoelectric actuator, an ultrasonic actuator, a shape memory alloy actuator and an ion-exchange resin actuator, which are made from, for example, polymers such as polymer gels and conductive polymers, ferroelectric substances, silicon or shape memory alloys, and which are deformed by stimulation of electricity, heat, light or the like.

For example, the ion-exchange resin actuator comprises an ion-exchange resin membrane and electrodes joined onto both surfaces of the ion-exchange resin membrane, and the ion-exchange membrane is curved (bent) and deformed by applying a potential difference to the membrane which is in a water-containing state (see Japanese Patent Laid-Open Publication No. 275078/1992).

Incidentally, medical equipment directly contacted with human bodies requires actuators made from soft materials doing no damage to tissues, and especially for artificial muscles such as artificial legs, artificial arms and artificial organs, actuators which are lightweight and make smooth motions are desired.

An attempt to use polyaniline (conductive polymer) for the actuators which are lightweight and make smooth motions has been proposed (see Applied Physics, Vol. 65, No. 8, pp. 803-810). The actuator using polyaniline has, for example, a three-layer structure wherein two polyaniline films are laminated onto both surfaces of an adhesive tape. If a voltage of 1.5 V is applied to the films on the both sides in an electrolitic solution, the film on the anode side extends and the film on the cathode side shrinks, whereby the actuator is curved.

The actuator using polyaniline, however, needs to be driven in an electrolitic solution, and hence there are many restrictions in the practical applications. For example, handling of the actuator is difficult, and the actuator does not work in air or pure water wherein no electrolyte is present. The actuator using polyaniline has other drawbacks such as small deformation, low degree of shrinkage and high rigidity.

Moreover, the polyaniline used for the actuator shows bad compatibility with other substances and has no melting point, so that processing of the polyaniline is difficult.

Accordingly, development of an artificial muscle which is flexible and lightweight, has quick response and is capable of undergoing large deformation is desired.

### OBJECT OF THE INVENTION

The present invention is intended to solve such problems associated with the prior art as mentioned above, and it is an object of the invention to provide an artificial muscle having quick response and capable of undergoing large deformation.

### SUMMARY OF THE INVENTION

The artificial muscle according to the present invention comprises a solid electrolyte molded product and polyaniline membranes which are formed on surface of the solid electrolyte molded product and are insulated from each other,
said artificial muscle being designed so that it can be freely deformed in the given direction by applying a potential difference to the polyaniline membranes to deform the polyaniline membranes.

By virtue of the above constitution, anions can enter and exit from the polyaniline membranes in the absence of an electrolyte, and there can be provided the artificial muscle which has excellent flexibility, is lightweight, has quick response, and can undergo large and strong deformation.

The artificial muscle according to the present invention may comprise a solid electrolyte molded product, polyaniline membranes which are formed on surface of the solid electrolyte molded product and are insulated from each other, and metallic electrodes provided on the polyaniline membranes, said artificial muscle being designed so that it can be freely deformed in the given direction by applying a potential difference between the metallic electrodes to deform the polyaniline membranes.

By virtue of the above constitution, a uniform potential can be applied to the whole polyaniline membranes, and therefore the response of the artificial muscle can be further quickened and the extent of deformation thereof can be increased.

In this artificial muscle, the metallic electrodes preferably are gold electrodes or platinum electrodes. When gold electrodes or platinum electrodes are provided, the response of the artificial muscle can be further quickened and the extent of deformation can be increased.

In the artificial muscle according to the present invention, the polyaniline membranes preferably comprise a modified polyaniline polymer obtained by graft modifying polyaniline with vinylsulfonic acid. When the polyaniline membranes comprise the modified polyaniline polymer, anions can more smoothly enter and exit from polyaniline, and hence the deformation force of the artificial muscle can be increased and the response thereof can be further quickened.

In the artificial muscle according to the present invention, the polyaniline membranes preferably contain a solid electrolyte. When the polyaniline membranes contain a solid electrolyte, anions can smoothly enter and exit from polyaniline, and the polyaniline membranes themselves can be softened. Therefore, the artificial muscle can be further improved in the flexibility, the deformation force and the response.

In the artificial muscle according to the present invention, the solid electrolyte molded product preferably is an anion-exchange resin molded product. When the solid electrolyte molded product is an anion-exchange resin molded product, the anion-exchange resin molded product is deformed in the same direction as that of the polyaniline membranes during deformation of the artificial muscle, and hence the deformation force and the extent of deformation of the artificial muscle can be further increased.

In the artificial muscle as described above, the polyaniline membranes preferably consist of plural pairs of polyaniline membranes. When the polyaniline membranes consist of plural pairs of polyaniline membranes, the artificial muscle can be deformed in the optional direction and becomes rotatable.

### BRIEF DESCRIPTION OF THE DRAWINGS

Fig. 1 is a schematic sectional view of a preferred embodiment of the artificial muscle according to the present invention when no voltage is applied.
Fig. 2 is a schematic sectional view of a preferred embodiment of the artificial muscle according to the present invention when a voltage is applied.
Fig. 3 is a schematic sectional view of another embodiment of the present invention when no voltage is applied.
Fig. 4 is a schematic sectional view of another embodiment of the present invention when a voltage is applied.

### DETAILED DESCRIPTION OF THE INVENTION

The embodiments of the present invention are described in detail hereinafter with reference to the attached drawings.

The artificial muscle of the present invention comprises a solid electrolyte molded product and polyaniline membranes which are formed on surface of the solid electrolyte molded product and are insulated from each other,
said artificial muscle being designed so that it can be freely deformed in the given direction by applying a potential difference to the polyaniline membranes to deform the polyaniline membranes.

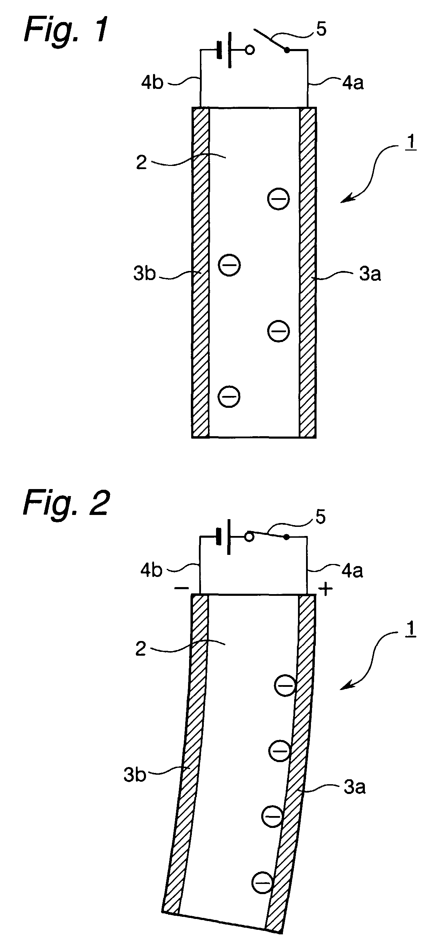

Fig. 1 and Fig. 2 are each a schematic sectional view of an embodiment of the artificial muscle of the present invention. In this embodiment, the artificial muscle 1 comprises a solid electrolyte molded product 2 having a shape of a slender rectangular flat plate and a pair of polyaniline membranes 3a, 3b which are formed on both surfaces of the solid electrolyte molded product and are insulated from each other, and this artificial muscle 1 is designed so that it can be curvedly deformed (curved or bent) by applying a voltage to the polyaniline membranes 3a, 3b to deform the polyaniline membranes.

To the polyaniline membranes 3a, 3b, ends of a pair of lead wires 4a, 4b are electrically connected, respectively, and the other ends of the lead wires 4a, 4b are connected to a power source 5.

The solid electrolyte molded product 2 is not limited to the rectangular flat plate in its shape, and it may be in a shape of a film, a column, a cylinder or the like.

Examples of the solid electrolytes for forming the solid electrolyte molded product 2 include anion-exchange resins, cation-exchange resins and amphoteric ion-exchange resins. Examples of the cation-exchange resins employable herein include those wherein functional groups such as sulfonic acid group and carboxyl group are introduced to resins such as polyethylene, polystyrene and fluororesins. Examples of the anion-exchange resins employable herein include those wherein functional groups such as amino group are introduced to resins such as polyethylene, polystyrene and fluororesins. In this embodiment, the anion-exchange resin is preferably employed. When the anion-exchange resin is employed, this anion-exchange resin is deformed in the same direction as that of the polyaniline membranes during deformation of the artificial muscle for the later-described reason, and consequently the extent of deformation and the deformation force of the artificial muscle can be increased.

The polyaniline membranes 3a, 3b comprise polyaniline represented by the following formula (I).

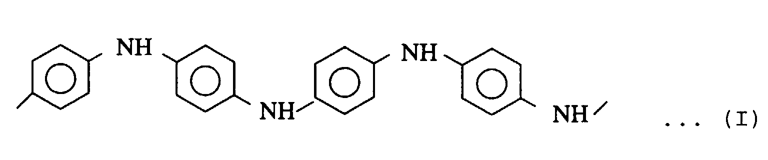

The polyaniline can be obtained by adding ammonium peroxodisulfate to a hydrochloric acid aqueous solution of aniline and polymerizing the aniline.

The polyaniline membranes 3a, 3b may be made of a modified polyaniline polymer obtained by graft copolymerization of polyaniline with vinylsulfonic acid. Derivatives of vinylsulfonic acids, such as salts (e.g., sodium salt, potassium salt) and esters thereof, are also available. The graft copolymerization can be carried out by irradiating polyaniline with electron rays and then immersing the polyaniline in a vinylsulfonic acid aqueous solution. The polyaniline represented by the formula (I) may be graft copolymerized with polyacrylic acid in place of the vinylsulfonic acid.

The polyaniline membranes 3a, 3b may contain a solid electrolyte. Examples of the solid electrolytes are the same as those used for forming the solid electrolyte molded product 2. The solid electrolyte contained in the polyaniline membranes may be the same as or different from that used for forming the solid electrolyte molded product 2.

The solid electrolyte is desirably contained in the polyaniline membranes in the range of 0.1 to 20 parts by weight, preferably 0.5 to 5 parts by weight, based on 100 parts by weight of the polyaniline.

The solid electrolyte can be introduced into the polyaniline membranes by adding a solution of the solid electrolyte to a solution of polyaniline, mixing them and drying the resulting mixture to remove the solvents. As the solvent to dissolve polyaniline, n-methylpyrrolidone is usually employed. As the solvent to dissolve the solid electrolyte, an alcohol is usually employed.

It is desirable that the solid electrolyte is homogeneously dispersed in the polyaniline membranes, because the extent of deformation of the artificial muscle can be increased. If the solid electrolyte is introduced into the polyaniline membranes, the artificial muscle can have lower rigidity and higher flexibility.

The polyaniline membranes 3a, 3b are provided on the solid electrolyte molded product 2 by coating the surfaces of the solid electrolyte molded product 2 with a solution of a solid electrolyte in an alcohol solvent or the like and then bonding the polyaniline membranes to the coated surfaces, respectively. The polyaniline membrane may be provided on the solid electrolyte molded product 2 in the following manner. The solid electrolyte molded product 2 having a solid electrolyte layer formed by coating the molded product surface with a solution of a solid electrolyte in an alcohol solvent or the like is immersed in a solution of polyaniline, then taken out and dried.

As the materials of the lead wires 4a, 4b, conductive materials such as copper, iron, aluminum, gold and platinum are employable. These materials can be subjected to plating or insulting coating, if desired.

In the artificial muscle of this embodiment, solid electrolyte layers may be provided on the surfaces of the polyaniline membranes 3a, 3b, if necessary. When the solid electrolyte layers are provided on the polyaniline membranes 3a, 3b, the later-described entrance and exit of anions into and from the polyaniline membranes undergoes not only from and into the solid electrolyte molded product but also from and into the solid electrolyte layers, and hence the response of the artificial muscle can be further quickened.

In this embodiment, a solid electrolyte molded product having a thickness of 0.15 mm, a width of 2 mm and a length of 30 mm is used as the solid electrolyte molded product 2, polyaniline membranes having a thickness of 5 to 20 µm are bonded to the both surfaces of the solid electrolyte molded product, and a direct current power source of 1.5 V is used as the power source 5.

The working principle of the artificial muscle is not clear, but it is presumed that upon application of a potential difference to the polyaniline membranes 3a, 3b, anions in the solid electrolyte molded product 2 move to the anode 3a from the cathode 3b as shown in Fig. 2, and the anions are inserted into the polymer chain of the polyaniline on the anode side to increase the bulk of the polymer chain of the polyaniline on the anode side. It is also presumed that the insertion of anions into the polymer chain of the polyaniline on the anode side increases electrostatic repulsion in the polyaniline molecules on the anode side and brings about structural change so that the polymer chain bent is made rigid and straight by the delocalization of the π electrons. It can be thought that, based on such principle, the polyaniline on the anode side extends and the polyaniline on the cathode side shrinks, whereby the artificial muscle is curved (see Applied Physics, Vol. 65, No. 8, pp. 803-810).

It is also presumed that if the polyaniline is graft modified with vinylsulfonic acid, the entrance and exit of anions smoothly proceeds owing to the graft chain, so that the deformation force of the artificial muscle can be increased and the response thereof can be further quickened.

It is further presumed that if an anion-exchange resin molded product is used as the solid electrolyte molded product 2, the anions in the anion-exchange resin move to the anode side, and together with the anions water molecules move in the anion-exchange resin, whereby a difference in the water content between the anode side and the cathode side of the anion-exchange resin is made to cause swelling of the resin on the anode side having a high water content and shrinkage of the resin on the cathode side having a low water content, and as a result, also the anion-exchange resin is curved. Accordingly, if the anion-exchange resin is used for the solid electrolyte molded product 2, the direction of the deformation in the polyaniline membranes 3a, 3b becomes equal to the direction of the deformation in the anion-exchange resin, and owing to their synergetic effects, deformation is promoted. That is, the deformation force of the artificial muscle can be increased.

The artificial muscle of the invention may comprise a solid electrolyte molded product, polyaniline membranes which are formed on surface of the solid electrolyte molded product and are insulated from each other, and metallic electrodes provided on the polyaniline membranes,
said artificial muscle being designed so that it can be freely deformed in the given direction by applying a potential difference between the metallic electrodes to deform the polyaniline membranes.

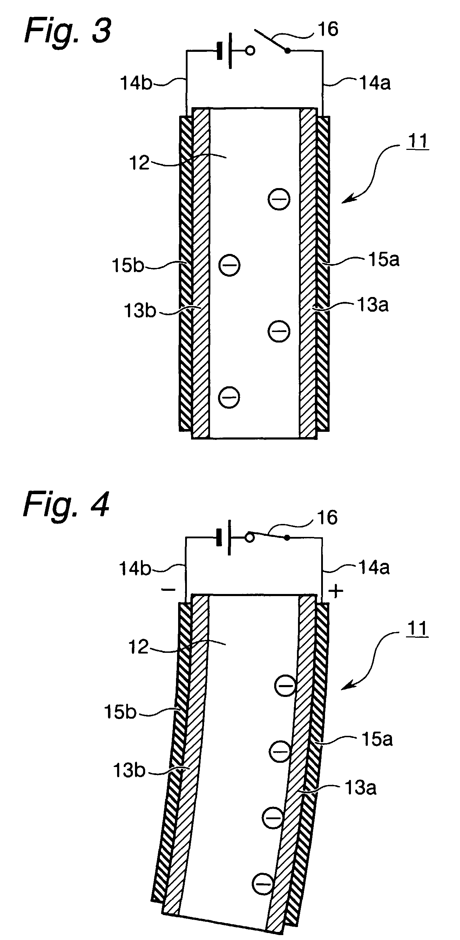

Fig. 3 and Fig. 4 are each a schematic sectional view of an embodiment of the above-mentioned artificial muscle. In this embodiment, the artificial muscle 11 comprises a solid electrolyte molded product 12 having a shape of a slender rectangular flat plate, a pair of polyaniline membranes 13a, 13b which are formed on both surfaces of the solid electrolyte molded product 12 and are insulated from each other, and metallic electrodes 14a, 14b provided on the polyaniline membranes, and this artificial muscle 11 is designed so that it can be freely deformed (curved or bent) in the given direction by applying a voltage between the metallic electrodes 14a, 14b to deform the polyaniline membranes.

To the metallic electrodes 14a, 14b, ends of a pair of lead wires 15a, 15b are electrically connected, respectively, and the other ends of the lead wires 15a, 15b are connected to a power source 16.

As the materials of the solid electrolyte molded product 12, the polyaniline membranes 13a, 13b and the lead wires 15a, 15b, the same materials as used for the solid electrolyte molded product 2, the polyaniline membranes 3a, 3b and the lead wires 4a, 4b in the aforesaid embodiment are employable.

The metallic electrodes 14a, 14b provided on the polyaniline membranes 13a, 13b preferably are gold electrodes or platinum electrodes. The metallic electrodes can be formed by conventional processes such as chemical plating, electroplating, vacuum deposition, sputtering, coating, press bonding and welding.

Also in this embodiment, a solid electrolyte molded product having a thickness of 0.15 mm, a width of 2 mm and a length of 30 mm is used as the solid electrolyte molded product 12, polyaniline membranes having a thickness of 5 to 25 µm are bonded to both surfaces of the solid electrolyte molded product 2, and a direct current power source of 1.5 V is used as the power source 16.

When the metallic electrodes are formed on the surfaces of the polyaniline membranes, the extent of deformation of the artificial muscle can be further increased and the response can be further quickened.

The artificial muscles of these embodiments as described above are generally used in the condition that the solid electrolyte molded product contains water. In the water, an electrolyte such as NaCl may be present. If the electrolyte is present, the deformation characteristics of the artificial muscle can be stabilized. If the solid electrolyte contains water, the artificial muscle can work even in pure water or air wherein no electrolitic solution is present around the polyaniline membranes.

In the artificial muscles of these embodiments, extension of one polyaniline membrane and shrinkage of the other polyaniline membrane are maintained while a constant voltage is applied, so that the deformed state can be fixed.

In the artificial muscles of these embodiments, further, the polyaniline membranes preferably consist of plural pairs of polyaniline membranes. When the plural polyaniline membranes consist of plural pairs of polyaniline membranes, the artificial muscle can be deformed in the optional direction and becomes rotatable.

### EFFECT OF THE INVENTION

Because of the constitution mentioned above, the artificial muscle of the invention can work in air or pure water wherein no electrolitic solution is present, and the artificial muscle is capable of undergoing large deformation and has high degree of shrinkage and excellent flexibility. When the metallic electrodes are provided on the polyaniline membrane surfaces, a uniform potential can be applied to the whole polyaniline membranes, and therefore the response of the artificial muscle can be further quickened and the extent of deformation thereof can be increased.

Accordingly, if the artificial muscle of the invention is connected to a tip of a guide means for a microdevice, the artificial muscles can be arbitrarily and positively curved (deformed) by operation of an operating controller. Therefore, as a result, in a microdevice attached to the tip of the guide means, such as surgical equipment for microsurgery, such as scissors, forceps, snare, laser knife and spatula, various sensors, and tools, the guidance can be improved, whereby the microdevices can be turned toward the desired place, and the turning operation can be rapidly and easily performed without skill.

If the artificial muscle of the invention is applied to surgical equipment such as tweezers, scissors, forceps, snare, laser knife, spatula and clip used for the microsurgery such as eye-ball operation, intra-cavity endoscopic operation and microvein suture operation, pain inflicted on the patients in the tests or treatments can be greatly eased, and physical or mental burden on the patients can be reduced.

If the artificial muscle of the invention is applied to various sensors or repair tools to inspect or repair facilities of plants such as power plant, and mechanical systems such as air craft engines or the like (particularly for piping system or engine interior), the inspection or the repairing can be surely performed without much labor or time.

In addition to the above, the artificial muscle of the invention can be favorably applied to high-frequency vibrating micropumps, health machines such as auxiliary power massage machine for rehabilitation, hygrometers, hygrometer controlling devices, soft manipulators, submerged valves, industrial machinery such as soft conveyor, underwater mobiles such as artificial goldfish and artificial seaweed, and hobby goods such as moving fishing bait and propulsive fin.

### EXAMPLE

The present invention is further described with reference to the following examples, but it should be construed that the invention is in no way limited to those examples.

### Example 1

A solid electrolyte molded product (Nafion 117, available from Dupont Co.) having a thickness of 0.15 mm, a width of 2 mm and a length of 30 mm was immersed in an alcohol solution of a solid electrolyte, then taken out and dried at room temperature, to form a solid electrolyte thin film on the solid electrolyte molded product.

Subsequently, the solid electrolyte molded product with the solid electrolyte thin film was immersed in an 8 % n-methylpyrrolidone solution of polyaniline, then taken out and dried at 60 °C for 5 hours, to form a polyaniline membrane on the solid electrolyte thin film.

Four sides of the thus treated solid electrolyte molded product were cut by a cutter to give divided polyaniline membranes insulated from each other. The polyaniline membranes were then immersed in an alcohol solution of a solid electrolyte and dried, to form a solid electrolyte layer covering the polyaniline membranes. Thus, an artificial muscle was prepared.

The polyaniline membranes of the artificial muscle were electrically connected to a power source through lead wires, and a voltage of 1.5 v was applied to the artificial muscle in distilled water. As a result, the tip of the artificial muscle moved toward the minus side by 2 mm.

### Example 2

An artificial muscle was prepared in the same manner as in Example 1, except that an anion-exchange resin was used as the solid electrolyte. A voltage of 1.5 V was applied to the artificial muscle in distilled water. As a result, the tip of the artificial muscle moved toward the minus side by 3.2 mm.

### Example 3

The solid electrolyte molded product having thereon a solid electrolyte thin film formed in the same manner as in Example 1 was immersed in a mixed solution (content of solid electrolyte component: 2 % by weight) of an 8 % n-methylpyrrolidone solution of polyaniline and a 5 % alcohol solution of a solid electrolyte, then taken out and dried at 60 °C for 5 hours, to form a polyaniline membrane on the solid electrolyte thin film.

The thus treated solid electrolyte molded product was cut in the same manner as in Example 1 to give divided polyaniline membranes insulated from each other, followed by the same operation as in Example 1, to prepare an artificial muscle. A voltage of 1.5 v was applied to the artificial muscle in distilled water. As a result, the tip of the artificial muscle moved toward the minus side by 2.8 mm.

### Example 4

A polyaniline powder was introduced into a vinyl plastic bag and spread thinly in the bag. The vinyl plastic bag is then passed in an electron ray irradiation device to irradiate the polyaniline with electron rays of 2 Mrad through the vinyl plastic bag.

After the electron ray irradiation, the polyaniline powder was added to a 10 % sodium p-styrenesulfonate aqueous solution, and graft reaction was carried out at 70 °C for 2 hours with stirring to obtain graft modified polyaniline.

The obtained graft modified polyaniline was dissolved in n-methylpyrrolidone to give an 8 % n-methylpyrrolidone solution of the graft modified polyaniline. Using this solution, an artificial muscle was prepared in the same manner as in Example 1. A voltage of 1.5 V was applied to the artificial muscle in distilled water. As a result, the tip of the artificial muscle moved toward the minus side by 3.1 mm.

### Comparative Example 1

On a slide glass plate horizontally placed, an 8 % n-methylpyrrolidone solution of polyaniline was spread in a uniform thickness, and then heated to 50 °C to evaporate the solvent, whereby a polyaniline film of 40 µm was obtained. Two of the polyaniline films were bonded with a double-sided adhesive tape to prepare an artificial muscle.

A voltage of 1.5 V was applied to the artificial muscle in an aqueous solution of 1 mol of HCl. As a result, the tip of the artificial muscle moved toward the minus side by 3.7 mm.

## Claims

1. An artificial muscle comprising a solid electrolyte molded product and polyaniline membranes which are formed on surface of the solid electrolyte molded product and are insulated from each other,
said artificial muscle being designed so that it can be freely deformed in the given direction by applying a potential difference to the polyaniline membranes to deform the polyaniline membranes.

2. An artificial muscle comprising a solid electrolyte molded product, polyaniline membranes which are formed on surface of the solid electrolyte molded product and are insulated from each other, and metallic electrodes provided on the polyaniline membranes,
said artificial muscle being designed so that it can be freely deformed in the given direction by applying a potential difference between the metallic electrodes to deform the polyaniline membranes.

3. The artificial muscle as claimed in claim 2, wherein the metallic electrodes are gold electrodes or platinum electrodes.

4. The artificial muscle as claimed in any one of claims 1 to 3, wherein the polyaniline membranes comprise a modified polyaniline polymer obtained by graft modifying polyaniline with vinylsulfonic acid.

5. The artificial muscle as claimed in any one of claims 1 to 4, wherein the polyaniline membranes are polyaniline membranes containing a solid electrolyte.

6. The artificial muscle as claimed in any one of claims 1 to 5, wherein the solid electrolyte molded product is an anion-exchange resin molded product.

7. The artificial muscle as claimed in any one of claims 1 to 6, wherein the polyaniline membranes consist of plural pairs of polyaniline membranes.
